# FASCICULE DE BREVET EUROPEEN

(11) **EP 4 138 777 B1**
(45) Date de publication et mention de la délivrance du brevet: **08.04.2026**
(21) Numéro de dépôt: 21719656.7
(22) Date de dépôt: 21.04.2021
(51) Int. Cl.: A61K 9/00, A61K 9/06, A61K 47/02, A61K 47/26, A61K 47/36, A61K 47/38, A61P 3/04

(54) **COMPOSITION D'HYDROGEL ASSIMILABLE PAR VOIE ORALE, KIT ET UTILISATION**
ORAL VERABREICHTE HYDROGELZUSAMMENSETZUNG, KIT UND VERWENDUNG
ORALLY ADMINISTERED HYDROGEL COMPOSITION, KIT AND USE

(30) Priorité: 21.04.2020 FR 2003982
(43) Date de publication de la demande: 01.03.2023
(73) Titulaire: Kifik, 13120 Gardanne (FR)
(72) Inventeur: HADJEBI, Ouadah, 13120 Gardanne France (FR); CHAIB-MEZRAG, Hassiba, 84700 Sorgues France (FR)
(74) Mandataire: Macquet, Christophe
(86) Numéro de dépôt international: PCT/EP2021/060435
(87) Numéro de publication internationale: WO 2021/214170

(56) Documents cités:
- WO-A1-2018/100340
- WO-A2-2007/039294
- WO-A2-2008/157318

## Description

### DOMAINE DE L'INVENTION

La présente invention concerne une composition d'hydrogel assimilable par voie orale, un kit comprenant une telle composition, et l'utilisation d'une telle composition pour le traitement d'individus en surpoids ou obèses, ou pour la délivrance d'actifs pharmaceutiques ou nutritionnels dans l'estomac d'un individu, de manière différée dans le temps.

### ART ANTERIEUR

La prévalence de l'obésité, qui est définie par un Indice de Masse Corporelle (IMC) > 30 kg/m², a doublé en 34 ans. L'Organisation Mondiale de la Santé (OMS) estime, qu'en 2016, 1,9 milliards d'adultes (de plus de 18 ans) et 41 millions d'enfants (de plus de 5 ans) étaient en surpoids ou obèses. Ces chiffres sont depuis en constante croissance. Il est estimé que, d'ici 2030, on devrait atteindre un niveau record de 3,3 milliards d'adultes en surpoids ou obèses. L'obésité a un impact sociétal et économique important. Elle est généralement associée à des complications graves voir fatales. On considère que, chaque minute dans le monde, plus de 5,3 personnes meurent des conséquences directes liées à l'obésité ou au surpoids.

Selon leur IMC, trois types de traitements sont proposés aux patients : le traitement médicamenteux, le traitement chirurgical et le traitement par pose d'un ballon gastrique.

Le traitement par pose d'un ballon gastrique est généralement destiné aux patients atteints d'obésité modérée à sévère avec un IMC supérieur à 30 et inférieur à 40 kg/m².

Ces ballons gastriques sont des dispositifs médicaux gonflables, généralement à base de silicone, qui peuvent être remplis soit par un gaz, un liquide, ou les deux. La procédure de placement d'un ballon est simple : il est placé dégonflé, par endoscopie, dans l'estomac d'un patient. Il est ensuite rempli au moyen d'un cathéter. La procédure de placement et de gonflage peut durer jusqu'à 20 minutes. Après gonflage, le cathéter est ensuite retiré.

A la fin de la période de traitement, qui dure 3 à 6 mois selon les ballons, le ballon est retiré, encore une fois, par voie endoscopique.

Toutefois, le traitement de patients au moyen de ballons gastriques selon l'art antérieur présentent différents inconvénients. En particulier, les procédures de placement et de retrait des ballons gastriques par voie endoscopiques sont invasives et présentent des dangers. Des complications assez graves, liées à la présence à long terme des ballons gastriques dans l'estomac, ont été rapportées. Il s'agit notamment d'altérations de la muqueuse gastrique, avec la formation d'ulcères et/ou la présence de perforations gastriques, de l'écrasement des organes sous-jacents occasionnant des pancréatites parfois aigues, ou d'obstructions intestinales dans le cas de migration des ballons. WO2007/039294 révèle les compositions pour l'emploi pour introduire la sensitivité de satiété, pour réduire l'appétit, pour traiter surpoids et obésité, de plus pour la libération de produits pharmaceutiques et nutraceutiques en utilisant les compositions gélifiantes. On peut trouver de poudre sec reconstitué dans l'eau contenant d'alginate de sodium, de carbonate de sodium, de carbonate de calcium, de glucono-delta lactone, de fructose et de bicarbonate de sodium. WO2018/100340 décrit un hydrogel contenant de l'eau, d'alginate, de glucono-delta-lactone et de microparticules qui contiennent de calcium inorganique et de gélatine recombinante. Dans WO 2008/157318 on peut trouver une composition gélifiante qui contient un agent gélifiant (alginate), 0.2-0.9 mM d'un cation divalent, 20-90% de calcium par 2mM d'entités d'acide glucuronique présent dans l'alginate.

### RESUME DE L'INVENTION

Compte tenu de ce qui précède, un problème que se propose de résoudre l'invention est notamment de fournir des moyens de traitement d'individus en surpoids ou obèses, qui évitent l'utilisation de ballons gastriques gonflables, qui nécessitent un placement dans l'estomac ou un retrait par voie endoscopique.

La solution de l'invention à ce problème posé a pour premier objet une composition d'hydrogel assimilable par voie orale comprenant :
un polymère d'alginate formant un gel en solution aqueuse, en présence d'un cation ;
un cation pour la polymérisation du polymère d'alginate en solution aqueuse ;
une solution aqueuse, en quantité suffisante ;
un agent de dissolution dudit polymère d'alginate dans la solution aqueuse ;
un agent retardateur de gélification ; et
un agent de flottaison pour former des bulles de CO₂ dans la composition d'hydrogel,
l'hydrogel formé au moyen de ladite composition d'hydrogel étant dissout au moyen d'un agent de dissolution final assimilable par voie orale, comme décrit dans la revendication 1.

De manière avantageuse : - la composition comporte en outre un agent de renforcement de la structure mécanique de l'hydrogel ; - la composition comporte en outre un agent radio-opaque ; - le polymère d'alginate est un polymère d'alginate de sodium, et en ce que le cation est le calcium ; - l'agent de dissolution du polymère d'alginate dans la solution aqueuse est le sucrose ; - l'agent retardateur de la gélification est le Na₂HPO₄ ; - l'agent de renforcement de la structure mécanique de l'hydrogel est choisi parmi le sorbitol, la spermine, le chitosan, l'agarose, le sodium dodécyl sulfate, la phosphatidylcholine, la cellulose microcristalline ; - l'agent radio-opaque est choisi parmi les composés comportant du Barium, par exemple BaSO₄ et les composés comportant de l'Iode ; - l'agent de dissolution final est choisi parmi les citrates, les chélateurs de calcium, par exemple le citrate de sodium, l'acide citrique ou l'EDTA ; - l'agent de flottaison est le CaCO₃, la glucono-δ-lactone ou un microorganisme ; la composition comprend 0,5 à 5% d'alginate de sodium de viscosité comprise entre 20-200mPa.s, 1 à 3% de CaSO4, 0,10 à 0,20% de Na₂HPO₄, 8 à 15% de sucrose, 2 à 8% de CaCO₃ ou 0,1 à 2% de levure, 0,5% à 8%de BaSO₄, et 0,5 à 8% de chitosan de viscosité comprise entre 10 et 50 mPa.s ou 0,5 à 8% de cellulose, les pourcentages étant des pourcentages massiques donnés en g/100 ml.

La solution de l'invention a pour deuxième objet une utilisation d'une composition telle que définie ci-dessus, pour le traitement d'individus en surpoids, qui présentent un Indice de Masse Corporelle supérieur ou égal à 25 kg/m².

De manière avantageuse : - l'utilisation est une utilisation pour le traitement d'individus obèses, qui présentent un Indice de Masse Corporelle supérieur ou égal à 30 kg/m².

La solution de l'invention a pour troisième objet une utilisation d'une composition telle que définie ci-dessus, pour la délivrance d'actifs pharmaceutiques ou nutritionnels dans l'estomac d'un individu, de manière différée dans le temps.

La solution de l'invention a pour quatrième objet comprenant une composition telle que définie ci-dessus, et un agent de dissolution assimilable par voie orale.

### BREVE DESCRIPTION DES FIGURES

L'invention sera mieux comprise à la lecture de la description non limitative qui suit, rédigée au regard des dessins annexés, dans lesquels :
- la figure 1 illustre, de manière schématique, les différentes étape pour le placement d'un hydrogel selon l'invention dans l'estomac d'un patient, ainsi que sa dissolution ;
- la figure 2 est composée de 4 photographies, les photographies A et C montrant une composition d'hydrogel et un hydrogel formé à partir de ladite composition, sans agent de renforcement, les photographies B et D montrant une composition d'hydrogel et un hydrogel formé à partir de ladite composition, avec agent de renforcement.
- la figure 3 illustre les résultats obtenus, en termes de temps de prise, de compositions d'hydrogel selon l'invention, selon les agents de renforcement qu'elle comporte ;
- la figure 4A illustre les différentes étapes mises en œuvre pour la détermination de la résistance d'hydrogels aux transitions de pH après l'ingestion d'un bol alimentaire ;
- la figure 4B présente les résultats obtenus de stabilité in vitro d'hydrogels selon l'invention comprenant un agent de renforcement ou non, après ingestion d'un bol alimentaire ;
- la figure 5A illustre les résultats obtenus, en termes de perte de poids, d'hydrogels selon l'invention, avec différents agents de renforcement ;
- la figure 5B illustre les résultats obtenus, en termes de perte de volume, d'hydrogels selon l'invention, avec différents agents de renforcement ;
- la figure 6 montre les résultats obtenus, en termes de perte de poids, d'hydrogels, dans le cadre d'une expérimentation in vitro visant à simuler une digestion artificielle en présente de chélateurs de calcium naturels ;
- la figure 7A illustre une première voie pour générer des bulles de CO₂ dans l'hydrogel selon l'invention, en utilisant le système dit de carbonate de calcium ;
- la figure 7B illustre une deuxième voie pour générer des bulles de CO₂ dans l'hydrogel selon l'invention, en utilisant le système dit NaHCO3 + Glucono-δ-lactone ;
- la figure 7C illustre une troisième voie pour générer des bulles de CO₂ dans l'hydrogel selon l'invention, en utilisant le système dit de levure/sucrose ;
- la figure 8A est une photographie qui illustre la flottaison d'un hydrogel selon l'invention, dans une solution acide simulant, *in vitro,* la solution acide d'un estomac humain ; et
- la figure 8B est une vue endoscopique de deux ballons d'un hydrogel selon l'invention, flottant dans un estomac d'un mini-porc.

### DESCRIPTION DETAILLEE DE L'INVENTION

L'invention concerne une composition d'hydrogel.

Cette composition est assimilable par voie orale par un individu ou un patient humain, sous la forme d'un sirop à boire. Cet individu est par exemple un individu adulte. Toutefois, il peut s'agir d'adolescents voire même d'enfants âgés d'au moins 5 ans.

La composition selon l'invention comprend un polymère d'alginate. Ce polymère d'alginate forme un gel en solution aqueuse, en présence d'un cation. Avantageusement, le polymère d'alginate est un polymère d'alginate de sodium, dont la polymérisation est initiée par le calcium. La source de calcium est par exemple fournie par du CaSO₄.

La solution aqueuse est par exemple de l'eau. Elle est présente, dans la solution, en quantité suffisante pour que l'hydrogel se forme.

La composition selon l'invention comporte en outre un agent de dissolution du polymère d'alginate dans la solution aqueuse.

La composition selon l'invention comporte par ailleurs un agent retardateur de gélification. Préférentiellement, l'agent retardateur de la gélification est le Na₂HPO₄.

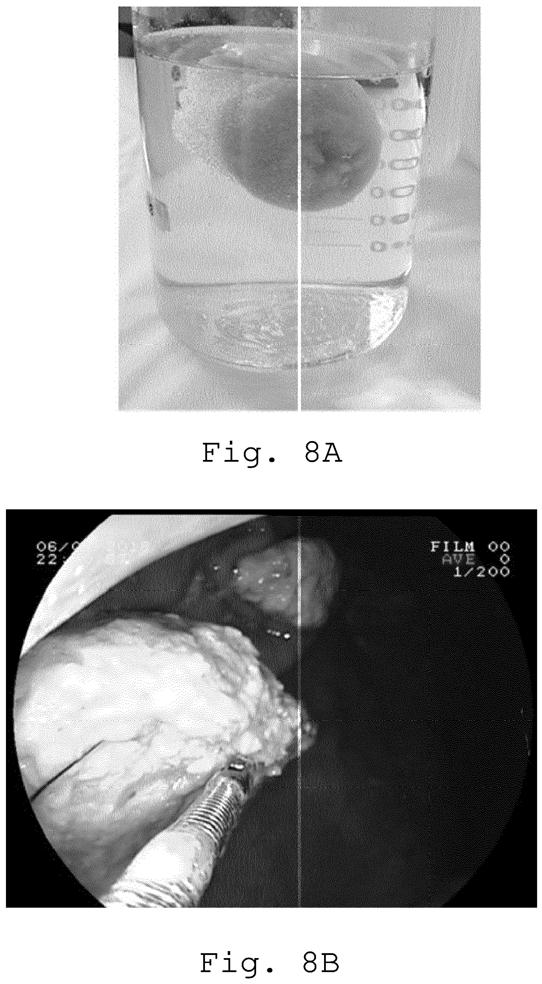

La composition selon l'invention comporte enfin un agent de flottaison. L'agent de flottaison est à l'origine de la formation de bulles de gaz dans la composition d'hydrogel. De manière avantageuse, l'agent de flottaison est le CaCO₃, la glucono-δ-lactone ou un microorganisme. La flottaison de l'hydrogel selon l'invention dans une solution acide simulant *in vitro* la solution acide d'un estomac humain est illustrée en figure 8A. Elle est en outre illustrée dans la vue endoscopique montrée à la figure 8B.

La composition selon l'invention comprend avantageusement un agent de renforcement. Cet agent de renforcement est un agent de renforcement de la structure mécanique de l'hydrogel. L'agent de renforcement est préférentiellement du type polymérique, formant des macromolécules qui sont incorporées dans l'hydrogel pour augmenter sa résistance mécanique. Préférentiellement, l'agent de renforcement de la structure mécanique de l'hydrogel est choisi parmi le sorbitol, la spermine, le chitosan, l'agarose, le sodium dodécyl sulfate, la phosphatidylcholine, la cellulose microcristalline.

La composition selon l'invention comprend aussi, de manière avantageuse, un agent radio-opaque. Préférentiellement, l'agent radio-opaque est choisi parmi les composés comportant du Barium, et les composés comportant de l'Iode. Plus préférentiellement, l'agent radio-opaque est BaSO₄.

Autrement dit, la composition selon l'invention comprend 0,5 à 5% d'alginate de sodium de viscosité comprise entre 20-200 mPa.s et/ou 1 à 3% de CaSO4 et/ou 0,10 à 0,20% de Na₂HPO₄ et/ou 8 à 15% de sucrose et/ou 2 à 8% de CaCO₃ ou 0,1 à 2% de levure et/ou 0,5% à 8% de BaSO₄ et/ou 0,5 à 8% de chitosan de viscosité comprise entre 10 et 50 mPa.s ou 0,5 à 8% de cellulose, les pourcentages étant des pourcentages massiques donnés en g/100 ml.

Selon l'invention, l'hydrogel formé au moyen de la composition d'hydrogel est dissout au moyen d'un agent de dissolution assimilable par voie orale. Cet agent de dissolution est dit agent de dissolution finale. Il est avantageusement choisi parmi les citrates, les chélateurs de calcium, par exemple l'acide phytique, l'acide oxalique, le citrate de sodium, l'acide citrique ou l'EDTA. La dissolution de l'hydrogel est avantageusement totale et ne donne pas lieu à des agrégats, en tant que produits de la dégradation.

Pour la mise en œuvre de l'invention, un kit est par exemple fourni à un patient, ou à un personnel de santé.

Ce kit comprend un premier récipient et un deuxième récipient.

Le premier récipient comporte les composés suivants sous forme de poudre : le polymère d'alginate, le composé formant un cation pour la polymérisation du polymère d'alginate, l'agent de dissolution du polymère d'alginate, l'agent retardateur de gélification, l'agent de flottaison, et, avantageusement, l'agent de renforcement, et l'agent radio-opaque.

Le deuxième récipient comprend l'agent de dissolution de l'hydrogel, de même sous forme de poudre.

Le contenu du premier récipient est dissout dans la solution aqueuse pour former la composition d'hydrogel.

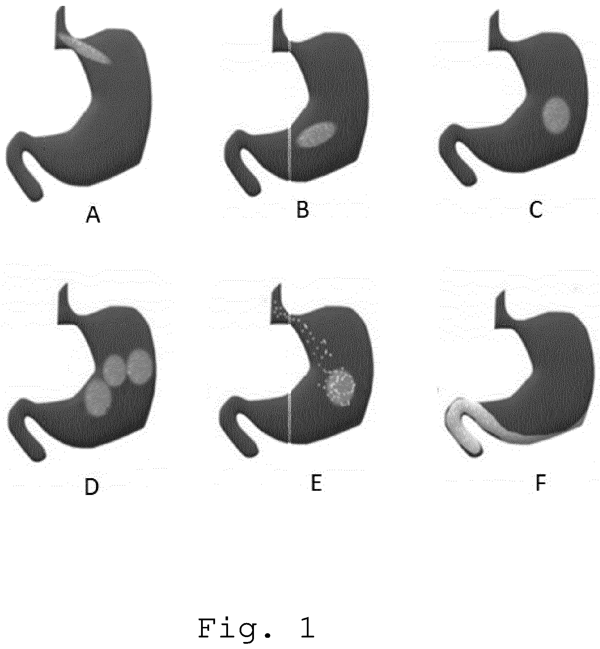

Ainsi que cela est montré à l'étape A de la figure 1, cette composition d'hydrogel est bue par le patient, après dissolution, à la manière d'un sirop. En pratique, l'agent retardateur retarde la polymérisation du polymère d'alginate. De ce fait, la composition est, à ce stade de la mise en œuvre de l'invention, buvable.

La composition assimilée par voie orale est alors amenée dans l'estomac du patient via l'œsophage.

L'agent retardateur de la polymérisation a une action temporaire. Ainsi que cela est montré à la figure 1, étape B, après l'admission de la composition d'hydrogel dans l'estomac, par exemple, de 2 à 3 minutes après, la composition fait l'objet d'une gélification dans l'estomac. En pratique le Calcium apporté par le CaSO₄ permet cette gélification.

L'estomac a un pH acide, qui varie dans le temps. Cette acidité est due à la présence d'acide chlorhydrique dans l'estomac. La gélification s'accompagne d'une réaction de l'agent de flottaison. Dans un exemple, l'agent de flottaison réagit avec l'acide chlorhydrique présent dans l'estomac pour former des bulles de gaz, à savoir du CO₂ dans la composition qui se gélifie. Les bulles de gaz formées sont emprisonnées dans le gel en formation. Ce gel est par suite un aérogel. Il peut être qualifié de gel hybride hydrogel/aérogel. Dans un autre exemple, l'agent de flottaison est formé de micro-organismes contenus dans la composition d'hydrogel. Les micro-organismes sont par exemple des levures, qui sont piégées dans le gel et produisent des bulles de CO₂ après avoir consommé le sucrose contenu également dans le gel. Elles réalisent une glycolyse du glucose en pyruvate avec libération de CO₂, ou une glycolyse du glucose en éthanol, en présence d'O₂.

Le gel ainsi formé, qui est montré à la figure 1, étape C, se présente sensiblement sous la forme d'un ballon sensiblement sphérique ou ovoïde. Le gel flotte dans l'estomac où il occupe une place qui est fonction de la quantité de composition admise dans l'estomac.

Pour la digestion, l'estomac se contracte puis se relaxe. L'agent de renforcement renforce la structure du gel selon l'invention. Sa présence permet de prolonger le temps de séjour de l'hydrogel à l'intérieur de l'estomac. Il lui permet de résister mécaniquement aux forces de contractions exercée par les couches musculaires de l'estomac.

Les gels d'alginate sont résistants en milieu acide, et ne sont pas dégradés par α-amylase humaine, contrairement aux gels de chitosan et aux gels d'amidon. Le gel selon l'invention, qui est établi dans l'estomac, est stable. Sa stabilité est conservée pendant plusieurs semaines ou mois.

Ainsi que cela est montré à la figure 1, étape D, de manière avantageuse, la composition d'hydrogel selon l'invention est prise séquentiellement sur une période de 3 semaines. Chaque semaine, le patient avale un volume déterminer de composition qui formera un ballon de 200 à 250 ml. Au total, 3 ballons de 200 à 250 ml seront présents dans l'estomac du patient. La prise séquentielle permet au patient de s'habituer à ressentir une masse dans l'estomac et de limite certains événements indésirables tels que les nausées, les vomissements et les douleurs abdominales.

Il est possible de vérifier le placement ainsi que la bonne tenue de l'hydrogel selon l'invention dans l'estomac du patient notamment grâce à la présence de l'agent radio-opaque, en procédant simplement à une radiographie de la zone abdominale du corps du patient où se situe l'estomac.

Pour l'élimination de l'hydrogel, ainsi que cela est montré à la figure 1, étape E, on utilise l'agent de dissolution contenu dans le deuxième récipient du kit selon l'invention.

Cet agent de dissolution est par exemple dissout dans une solution aqueuse. Il est ensuite bu par le patient. La solution comportant cet agent est ensuite amenée dans l'estomac via le duodénum. Une fois en contact avec l'hydrogel de l'invention, elle le dissout et celui-ci est évacué de l'estomac du patient lors de la vidange gastrique. Cette dernière étape est notée F à la figure 1.

Selon l'invention, la composition d'hydrogel est ainsi susceptible d'être utilisée pour le traitement d'individus en surpoids, qui présentent un Indice de Masse Corporelle supérieur ou égal à 25 kg/m2. De manière avantageuse, elle est utilisée pour le traitement d'individus obèses, qui présentent un Indice de Masse Corporelle supérieur ou égal à 30 kg/m².

Selon l'invention, la composition d'hydrogel peut être utilisée pour la délivrance d'actifs pharmaceutiques ou nutritionnels dans l'estomac d'un individu, de manière différée dans le temps. Les actifs sont alors avantageusement contenu dans la composition d'hydrogel, puis emprisonnés dans celui-ci, et leur relargage dans l'estomac est différé.

En définitive, l'invention concerne un dispositif intragastrique de classe III innovant capable de réduire à la fois les problèmes de sécurité liés au ballon gastrique mais aussi les coûts. Il a préférentiellement été développé pour des adultes dont l'IMC est compris entre 30 et 40 kg/m², mais peut être, à terme, proposé à des adolescents ou enfants. La formulation de la composition de gel selon l'invention est unique, et composée d'agents biocompatibles. Aucun agent toxique n'est utilisé. La composition est finalement administrée par voie orale, sous la forme d'un sirop. Elle forme une structure sphérique ou ovoïde, aérée grâce à la présence de bulles, radio-opaque lorsqu'elle est en présence du suc gastrique notamment à des pH entre 2 et 3. Cette structure est stable pendant plus de 4 mois dans l'environnement intragastrique simulé. Elle conserve 80% de son poids/volume à la fin du traitement. Elle peut être entièrement dissoute, sans former d'agrégats au bout de quelques heures via la deuxième solution, de même aqueuse, administrée aussi par voie orale et composée également d'un additif alimentaire.

### EXEMPLE 1 : PREPARATION D'UN HYDROGEL

La composition d'hydrogel selon l'invention suivante a été préparée, dans lequel les pourcentages sont donnés en poids par rapport au volume p/v :

| | |
|---|---|
| Na-Alginate | 2% |
| CaSO₄ | 1,75% |
| Na₂HPO₄ | 0,16% |
| sucrose | 12% |
| CaCO₃ | 1% |
| Eau | 83,09% |

Pour la préparation de cet hydrogel, tous les ingrédients ont été mélangés sous forme de poudre dans un bécher puis de l'eau a été ajoutée pour l'obtention d'un volume d'hydrogel de 250 mL correspondant à un ballon assimilé en une prise par un patient.

### EXEMPLE 2 : AUTRE EXEMPLE DE COMPOSITION D'HYDROGEL

La composition d'hydrogel selon l'invention suivante a été préparée, dans lequel les pourcentages sont donnés en poids par rapport au volume p/v :

| | |
|---|---|
| Na-Alginate | 2% |
| CaSO₄ | 1,75% |
| Na₂HPO₄ | 0,14% |
| Sucrose (D+) | 12% |
| CaCO₃ | 5% |
| Chitosan | 0,5 - 1% |
| Cellulose | 0,5 - 1% |
| BaSO₄ | 5% |
| Eau | q.s. |

### EXEMPLE 2 : AGENT DE RENFORCEMENT DE L'HYDROGEL

On notera que la littérature scientifique est pauvre en ce qui concerne les forces de compressions rencontrées à l'intérieur de la lumière de l'estomac humain. Selon un premier document, ces forces ne dépassent pas 13 kPa. Selon un deuxième document, pendant la digestion, ces forces varient entre 5 kPa et 67 kPa. Selon un troisième document, elles sont en moyenne de 96 ± 12 Pa pour un estomac humain nourri. Les propriétés mécaniques de l'hydrogel selon l'invention ont été évaluées par des essais de compression statique à l'aide d'une machine de mesure de la résistance à la compression de matériaux Lloyd^{™} LRX PLUS. Au préalable, les paramètres de cette machine ont été optimisés en fonction des propriétés des gels et, notamment, des dimensions des gels, de la plage de forces, du taux de déformation, de la déformation maximale. Une précharge de 0,5 N et une vitesse de compression de 10 mm/min ont été sélectionnées.

L'hydrogel de l'exemple 1 a été testé. Avant rupture, ce gel, qui ne comporte pas d'agent de renforcement, est capable de supporter une contrainte moyenne de 1342 ± 50 Pa correspondant à une déformation moyenne de 26 ± 9% de sa longueur.

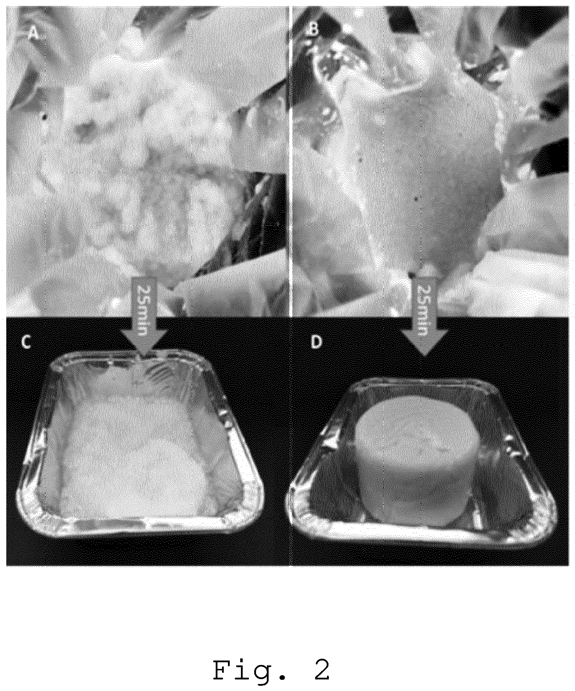

Les photos de la figure 2 montrent, à gauche, photographie C, un gel obtenu selon la composition d'hydrogel de l'invention selon l'Exemple 1, sans agent de renforcement (le chitosan) et, à droite, photographie D, un gel ayant la même composition, mais comportant du chitosan en tant qu'agent de renforcement. Ainsi que cela apparaît sur ces photos, l'agent de renforcement permet de maintenir le gel final sous la forme souhaitée, après 25 min.

Les agents de renforcement contenus dans le tableau ci-dessous ont été introduits dans le gel de l'exemple 1 à de concentrations allant de 0,1 à 20 % p/v. Un résumé des gels produits et leurs données de contrainte / déformation se trouvent dans le tableau ci-dessous.

**[Table 1]**

| Agent de renforcement | Concentration (% p/v) | Pression max avant rupture (Pa) | Tension max avant rupture (%) | Formation d'un gel ? |
|---|---|---|---|---|
| Sorbitol | 12 | 892 | 18 | Oui - 2 phases |
| Spermine | 0,1 | | | Non |
| | 1 | 1409 | 24 | Oui |
| Chitosan (Haute viscosité) | 0,1 | | | Non |
| | 1 | 4361 | 28 | Oui |
| Chitosan (Faible viscosité) | 0,1 | | | Non |
| | 1 | 1673 | 23 | Oui |
| | 5 | 9499 | 32 | Oui |
| | 10 | 6322 | 29 | Oui |
| Agarose | 1 | 1678 | 25 | Oui |
| | 5 | 8287 | 35 | Oui |
| | 10 | 7549 | 32 | Oui |
| Cellulose | 10 | 12273 | 41 | Oui |
| Barium sulfate | 5 | 3886 | 26 | Oui |
| | 20 | 6182 | 24 | Oui |
| | 80 | 5961 | 27 | Oui |
| Carbonate de calcium | 20 | 4362 | 28 | Oui |
| Sodium Dodecyl Sulfate (SDS) | 0,1 | 1348 | 26 | Oui |
| | 1 | 2773 | 23 | Oui |
| Phosphatidylcholine | 0,1 | 658 | 14 | Oui |
| | 10 | 833 | 21 | Oui |
| | 10 | 1314 | 22 | Oui |
| Chitosan LV - sulfate de Barium | 5 - 5 | 9126 | 26 | Oui |

### EXEMPLE 3 : TEMPS DE GELIFICATION DES HYDROGELS COMPRENANT UN AGENT DE RENFORCEMENT

Dans cet exemple, on a déterminé les temps de gélification pour des compositions de gel d'alginate comprenant un agent de renforcement, et qui sont capables de résister à une contrainte maximale d'au moins 4000 Pa. Ce temps de gélification ne devrait pas être inférieur à 5 min de manière que le patient ait le temps de boire la composition selon l'invention, et que celle-ci soit admise dans l'estomac. Ces compositions sont celles de l'exemple 2, qui comprennent le chitosan HV (0,1 et 1% p/v) et LV (0,1, 1, 5 et 10% p/v), l'agarose (1, 5 et 10% p/v), la cellulose (10% p/v), le sulfate de baryum (1, 5, 10 et 20% p/v), le calcium carbonate (20% p/v) et le chitosan LV - sulfate de baryum (tous deux à 5% p/v). Dans un premier temps, le temps de prise de ces gels renforcés a été déterminé. A cet effet, une fois mélangées, les poudres sont ajoutées à de l'eau distillée puis la solution est agitée à la spatule pendant quelques secondes . La dispersion aqueuse est agitée à température ambiante sur une plaque à bascule réglée à 10 tr/min. Toutes les minutes, le récipient est incliné à 90° pour vérifier si la solution coule toujours ou non. Les mesures sont arrêtées après 25 minutes. Cette limite de temps a été choisie en tenant compte du fait que la moitié du temps de vidange de l'estomac après ingestion d'eau, est de 13 ± 1 min.

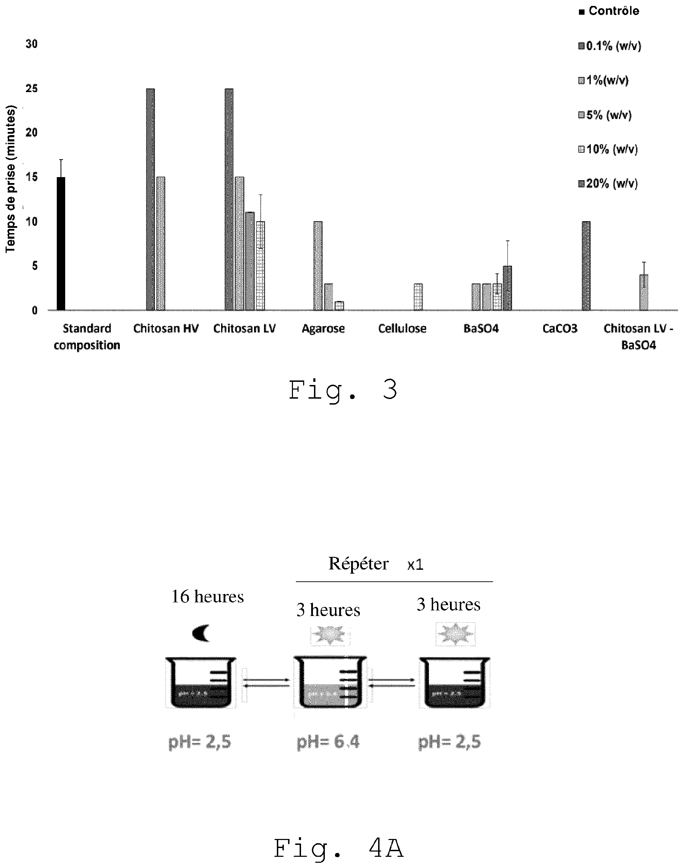

Les résultats sont montrés à la figure 3. Ainsi que cela apparaît à cette figure, le chitosan, en particulier LV, présente l'intérêt le plus grand car il a peu d'impact sur le temps de prise et donne une bonne résistance mécanique. Le carbonate de calcium a également peu d'impact sur la cinétique de gélification mais favorise moins de renforcement mécanique. Au contraire, la cellulose et le sulfate de baryum induisent une résistance élevée aux contraintes/déformations mais déclenchent une gélification rapide. Le sulfate de baryum est également intéressant pour ses capacités d'agent de contraste pour la radioscopie et la tomodensitométrie. Enfin, un composite à base de chitosan LV et de sulfate de baryum possède de bonnes propriétés mécaniques mais forme encore du gel trop rapidement. Cependant, comme elle combine les propriétés mécaniques du chitosan et les propriétés de contraste du sulfate de baryum, ce composite pourrait être un bon compromis.

### EXEMPLE 4 : STABILITE DES HYDROGELS IN VITRO ET IMPACT DE L'AGENT DE RENFORCEMENT

Des hydrogels selon l'invention ont été placés pendant 4 mois dans un suc gastrique simulé oscillant d'un pH extrêmement acide, à pH = 2,4 pendant 3h00 ou 16h00, à un pH quasi-neutre égal à 6,4 pendant 3h00 ainsi que cela est schématisé à la figure 4A. Deux transitions par jour (3h00 -> 3h00 -> 3h00 -> 3h00 -> 16h00) ont été réalisées pendant 4 mois consécutifs.

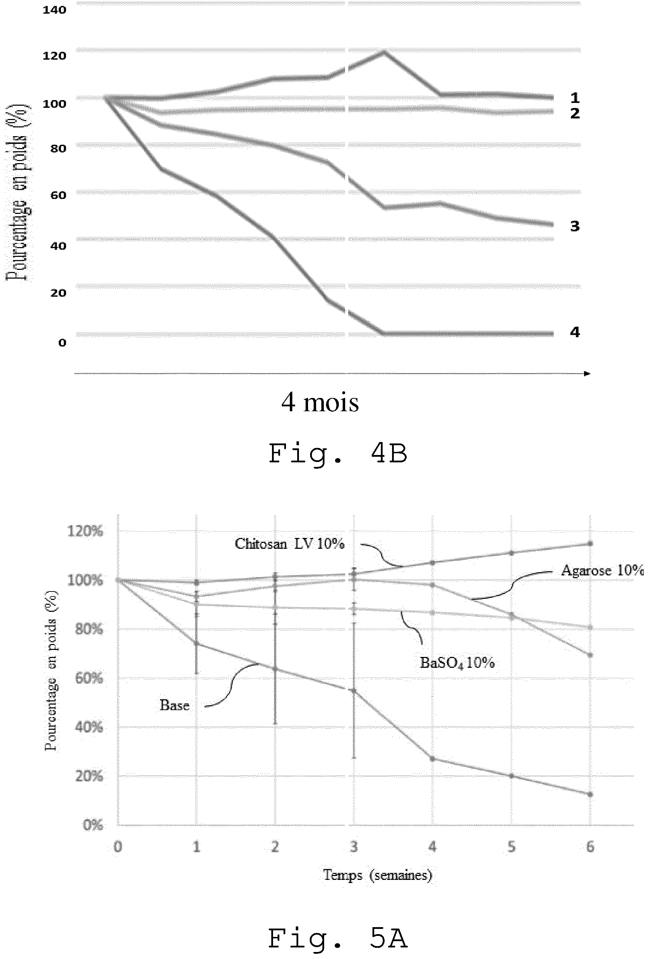

A la figure 4B, qui illustre les résultats obtenus en termes de perte de poids en pourcents selon le temps, l'hydrogel 1 est un hydrogel comportant le polymère d'alginate polymérisé par le cation en solution aqueuse en présence d'un agent de renforcement. L'hydrogel 2 est l'hydrogel de l'Exemple 2, comprenant un agent de renforcement. L'hydrogel 3 est formé d'un alginate polymérisé, sans agent de renforcement, comportant un agent radio-opaque, à savoir le BaSO4. L'hydrogel 4 est formé d'un alginate polymérisé, sans agent de renforcement et sans agent radio-opaque.

Ainsi que cela apparaît sur les courbes montrées à la figure 4B, seuls les hydrogels 1 et 2 comportant un agent de renforcement présentent un résistance dans le temps à peu près constante pendant les 4 mois de l'expérimentation.

En l'absence de tel agent, la stabilité de l'hydrogel n'est pas assurée dans le temps.

### EXEMPLE 5 : STABILITE DES HYDROGELS SELON LE PH

La stabilité physico-chimique d'hydrogels selon l'invention dans un liquide gastrique simulé (pH 2,5 et 6,4) a été évaluée en mesurant leur poids et leur volume chaque semaine pendant une période de six semaines. Quatre compositions de gel ont été comparées dans cette étude : la composition de base de l'exemple 1, ainsi que les compositions comportant du chitosan LV 10% p/v, de l'agarose 10% p/v, du BaSO4 10% (p/v).

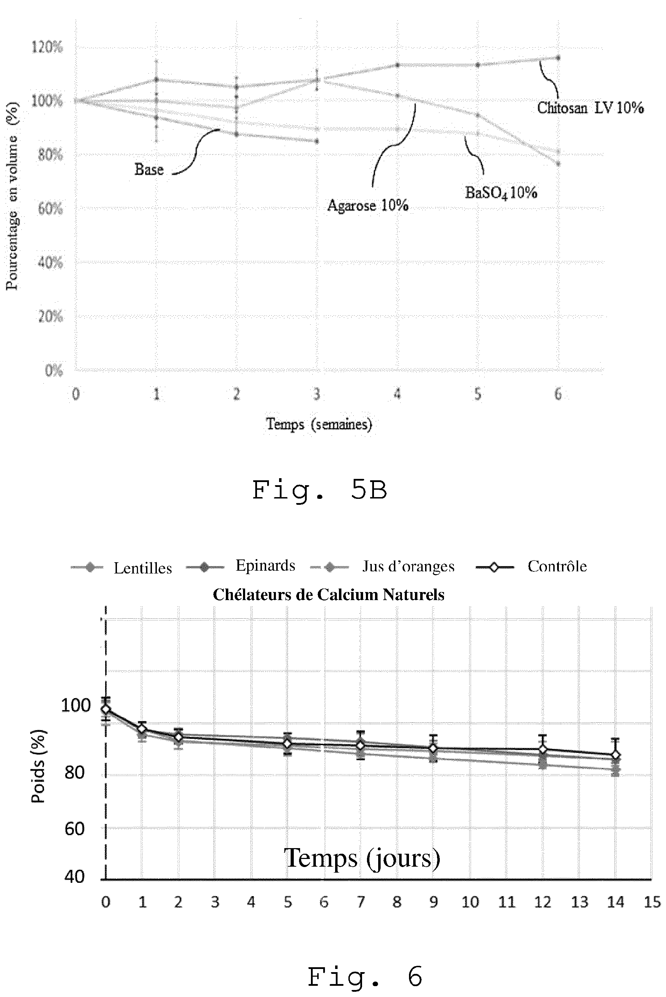

Ainsi que cela est montré aux figures 5A et 5B, la composition de l'Exemple 1 (10% p/v) montre une décroissance rapide mais variable en termes de poids et une décroissance plus lente du volume. Cette évolution temporelle correspond à la capacité de flottement de ces gels. La composition comprenant du chitosan LV (10% p/v) présente de l'intérêt, et on constate que le poids et le volume augmentent lentement avec le temps. Toutefois, l'hydrogel ainsi formé n'est pas capable de flotter. L'hydrogel comportant de l'agarose (10% p/v) est quelque peu stable pendant quatre semaines avant qu'une dégradation en termes de poids et de volume ne soit observée. Ce gel n'est pas capable de flotter, tout comme le gel précédent. L'hydrogel comportant du sulfate de barium (10% p/v) présente une décroissance lente et constante de son poids et de son volume et, de même que les autres gels, il n'est pas capable de flotter.

### EXEMPLE 6 : STABILITE EX-VIVO ET IMPACT DE L'AGENT DE RENFORCEMENT : DIGESTION ARTIFICIELLE IN VITRO

La composition d'hydrogel selon l'Exemple 2 a fait l'objet d'essais de stabilité au moyen de tests de digestion artificielle *in vitro.* Une quantité d'hydrogel correspondant à un volume de 50 mL a été placée pendant 14 jours consécutifs à 37°C sous agitation dans un tampon de digestion, à pH = 3, avec ou sans aliments contenant de fortes teneurs en chélateurs de calcium, à savoir des lentilles, qui contiennent de l'acide phytique, des épinards, qui contiennent de l'acide oxalique, ou du jus d'oranges, qui contient de l'acide citrique. Ainsi que cela apparaît à la figure 6, l'hydrogel selon l'invention présente une parfaite résistance aux conditions extrêmes liées à la digestion (pH acide et chélateurs de calcium naturels apportés par la nourriture), le contrôle étant réalisé lorsque ledit hydrogel ne comporte pas de chélateurs de calcium.

### EXEMPLE 7 : FLOTTABILITE DE L'HYDROGEL

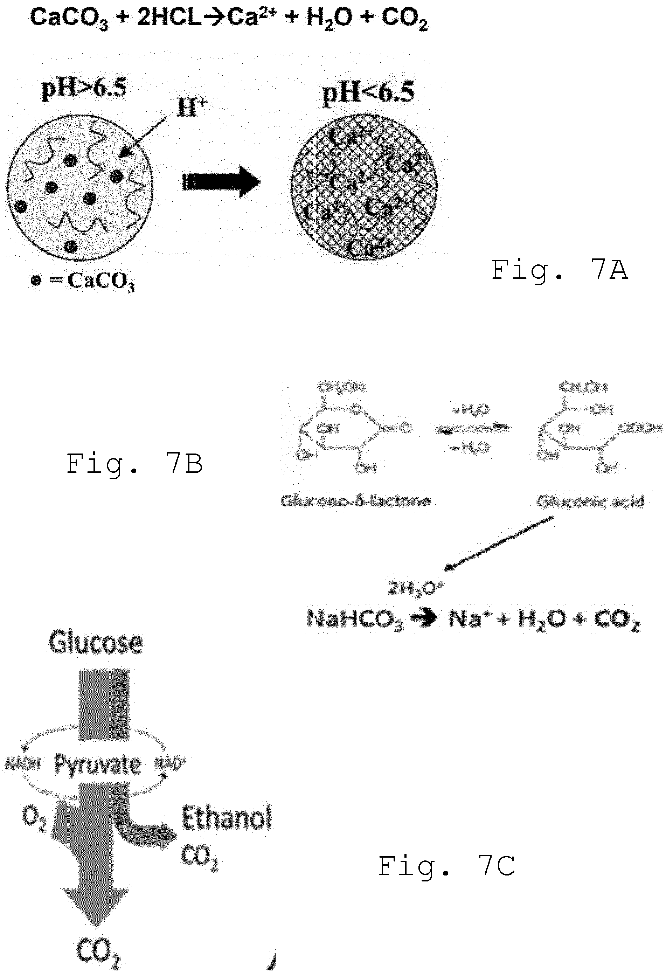

Les capacités moussantes et gonflantes des hydrogels selon l'invention sont liées à la teneur et à la réactivité des agents de flottaison. Le carbonate de calcium a été utilisé avec succès pour produire du CO₂ et des aérogels. En adoptant la composition de base de l'exemple 1, un milieu acide s'est révélé suffisant pour déclencher la dissolution du carbonate de calcium et le flottement des gels. Alternativement, le système carbonate de calcium peut être remplacé soit par un système gluconolactone-bicarbonate de sodium, soit par un système levure-saccharose. Les trois systèmes sont présentés aux figures 7A, 7B et 7C.

La figure 7A est une photographie qui montre le gel obtenu selon la composition de l'exemple 1, dans une solution acide comprenant de l'HCl. Ainsi que cela est montré dans cette figure, le gel flotte, *in vitro,* dans cette solution acide. La figure 5B est une vue endoscopique de deux ballons de ce même gel, *in vivo,* dans l'estomac d'un mini-porc, 1 semaine après l'ingestion de la composition de l'hydrogel. De même, l'hydrogel ou, en fait, l'hybride hydrogel-aérogel, flotte dans l'estomac de ce mini-porc.

Le système carbonate de calcium est simple de mise en œuvre. Il est sûr. Il a été étudié avec des hydrogels comportant du chitosan comme agent de renforcement. Tous les gels testés se sont révélés être mécaniquement stables. Cependant, à pH 2,5, après un jour, aucun d'entre eux n'a pu flotter, même si certaines bulles ont pu être observées à leur surface. Par contre, juste après leur incubation dans un milieu acidifié à pH 1,2, tous les échantillons testés ont pu rapidement flotter. Après 7 jours, aucun changement n'a été observé.

Le système gluconolactone-bicarbonate de sodium illustré à la figure 7B est basé sur l'hydrolyse de la lactone pour produire un l'acide gluconique réagissant à son tour avec le bicarbonate de sodium pour produire du CO₂. Ce système a été testé avec des gels d'alginate renforcés avec du chitosan. Il apparaît que les gels renforcés au chitosan ne sont pas sensiblement affaiblis par le système moussant et flottent pour certains dès le premier jour d'incubation à pH 2,5. La présence de chitosan améliore les propriétés élastiques des gels et prévient l'affaiblissement et la destruction induits par l'expansion gazeuse. Le gonflement des gels est inversement proportionnel à la concentration de chitosan.

Le système levure-saccharose (Saccharomyces Cerevisiae/sucrose) est un système couramment utilisé en boulangerie pour garantir l'expansion de la pâte à pain avant de procéder à sa solidification par la cuisson. Selon ce processus de bio-fermentation, le CO₂ est produit par la consommation de saccharose suivie d'une glycolyse aérobie ou anaérobie. Ce système a été testé avec des gels d'alginate renforcés par du chitosan. Pour toutes les compositions les gels sont mécaniquement stables probablement grâce à l'action de réticulation du chitosan. Dès le premier jour, certaines compositions testées, qui contiennent 0,6 et 0,9% (p/v) de levure sèche sont en mesure de générer suffisamment de gaz pour constituer un aérogel. Après 3 jours d'incubation, des hydrogels contenant 0,3% (p/v) de levure sèche, ont également été capable de flotter.

## Revendications

1. Composition d'hydrogel assimilable par voie orale comprenant :
un polymère d'alginate formant un gel en solution aqueuse, en présence d'un cation ;
un cation pour la polymérisation du polymère d'alginate en solution aqueuse ;
une solution aqueuse, en quantité suffisante ;
un agent de dissolution dudit polymère d'alginate dans la solution aqueuse ;
un agent retardateur de gélification ;
un agent de flottaison pour former des bulles de CO₂ dans la composition d'hydrogel ; et
un agent de renforcement de la structure mécanique de l'hydrogel,
l'hydrogel formé au moyen de ladite composition d'hydrogel étant dissout au moyen d'un agent de dissolution final assimilable par voie orale.

2. Composition selon la revendication 1, **caractérisée en ce que** l'agent de renforcement est du type polymérique, formant des macromolécules incorporées dans l'hydrogel.

3. Composition selon l'une des revendications 1 ou 2, **caractérisée en ce qu'**elle comporte en outre un agent radio-opaque.

4. Composition selon l'une des revendications 1, 2 ou 3, **caractérisée en ce que** le polymère d'alginate est un polymère d'alginate de sodium, et **en ce que** le cation est le calcium.

5. Composition selon l'une des revendications précédentes, **caractérisée en ce que** l'agent de dissolution du polymère d'alginate dans la solution aqueuse est le sucrose.

6. Composition selon l'une des revendications précédentes, **caractérisée en ce que** l'agent retardateur de la gélification est le Na₂HPO₄.

7. Composition selon l'une des revendications précédentes, **caractérisée en ce que** l'agent de renforcement de la structure mécanique de l'hydrogel est choisi parmi le sorbitol, la spermine, le chitosan, l'agarose, le sodium dodécyl sulfate, la phosphatidylcholine, la cellulose microcristalline.

8. Composition selon l'une des revendications précédentes, **caractérisée en ce que** l'agent radio-opaque est choisi parmi les composés comportant du Barium, par exemple BaSO₄ et les composés comportant de l'Iode.

9. Composition selon l'une des revendications précédentes, **caractérisée en ce que** l'agent de dissolution final est choisi parmi les citrates, les chelateurs de calcium, par exemple le citrate de sodium, l'acide citrique ou l'EDTA.

10. Composition selon l'une des revendications précédentes, **caractérisé en ce que** l'agent de flottaison est le CaCO₃, la glucono-δ-lactone ou un microorganisme.

11. Composition selon l'une des revendications précédentes, **caractérisée en ce qu'**elle comprend 0,5 à 5% d'alginate de sodium de viscosité comprise entre 20-200mPa.s, 1 à 3% de CaSO4, 0,10 à 0,20% de Na₂HPO₄, 8 à 15% de sucrose, 2 à 8% de CaCO₃ ou 0,1 à 2% de levure, 0,5% à 8% de BaSO₄, et 0,5 à 8% de chitosan de viscosité comprise entre 10 et 50 mPa.s ou 0,5 à 8% de cellulose, les pourcentages étant des pourcentages massiques donnés en g/100 ml.

12. Utilisation d'une composition selon l'une des revendications précédentes, pour le traitement d'individus en surpoids, qui présentent un Indice de Masse Corporelle supérieur ou égal à 25 kg/m2.

13. Utilisation selon la revendication 12, pour le traitement d'individus obèses, qui présentent un Indice de Masse Corporelle supérieur ou égal à 30 kg/m².

14. Utilisation d'une composition selon l'une des revendications 1 à 11, pour la délivrance d'actifs pharmaceutiques ou nutritionnels dans l'estomac d'un individu.

15. Kit comprenant une composition selon l'une des revendications 1 à 11, et un agent de dissolution du assimilable par voie orale.

## Patentansprüche

1. Oral assimilierbare Hydrogelzusammensetzung, umfassend:
ein Alginatpolymer, das in wässriger Lösung ein Gel bildet, in Gegenwart eines Kations;
ein Kation zur Polymerisation des Alginatpolymers in wässriger Lösung;
eine wässrige Lösung in ausreichender Menge;
ein Lösungsmittel des Alginatpolymers in der wässrigen Lösung;
ein gelierverzögerndes Mittel;
ein Auftriebsmittel zur Bildung von CO₂-Blasen in der Hydrogelzusammensetzung; und
ein Verstärkungsmittel der mechanischen Struktur des Hydrogels,
wobei das aus der Hydrogelzusammensetzung gebildete Hydrogel mittels eines oral assimilierbaren Lösungsmittels aufgelöst wird.

2. Zusammensetzung nach Anspruch 1, **dadurch gekennzeichnet, dass** das Verstärkungsmittel vom polymeren Typ ist und Makromoleküle bildet, die in das Hydrogel eingebettet sind.

3. Zusammensetzung nach einem der Ansprüche 1 oder 2, **dadurch gekennzeichnet, dass** sie ferner ein röntgendichtes Mittel umfasst.

4. Zusammensetzung nach einem der Ansprüche 1, 2 oder 3, **dadurch gekennzeichnet, dass** das Alginatpolymer ein Natriumalginatpolymer ist, und dass das Kation Kalzium ist.

5. Zusammensetzung nach einem der vorstehenden Ansprüche, **dadurch gekennzeichnet, dass** das Lösungsmittel des Alginatpolymers in der wässrigen Lösung die Saccharose ist.

6. Zusammensetzung nach einem der vorstehenden Ansprüche, **dadurch gekennzeichnet, dass** das Verzögerungsmittel der Gelierung das Na₂HPO₄ ist.

7. Zusammensetzung nach einem der vorstehenden Ansprüche, **dadurch gekennzeichnet, dass** das Verstärkungsmittel für die mechanische Struktur des Hydrogels aus Sorbit, Spermin, Chitosan, Agarose, Natriumdodecylsulfat, Phosphatidylcholin und mikrokristalliner Cellulose ausgewählt ist.

8. Zusammensetzung nach einem der vorstehenden Ansprüche, **dadurch gekennzeichnet, dass** das röntgendichte Mittel aus Verbindungen ausgewählt ist, die Barium aufweisen, beispielsweise BaSO₄, und Verbindungen, die Jod aufweisen.

9. Zusammensetzung nach einem der vorstehenden Ansprüche, **dadurch gekennzeichnet, dass** das endgültige Lösungsmittel aus Citraten, Calciumchelatoren, beispielsweise Natriumcitrat, Zitronensäure oder EDTA, ausgewählt ist.

10. Zusammensetzung nach einem der vorstehenden Ansprüche, **gekennzeichnet dadurch, dass** das Auftriebsmittel CaCO₃ Glucono-δ-lacton oder ein Mikroorganismus ist.

11. Zusammensetzung nach einem der vorstehenden Ansprüche, **dadurch gekennzeichnet, dass** sie 0,5 bis 5 % Natriumalginat mit einer Viskosität zwischen 20 und 200 mPa.s, 1 bis 3 % CaSO4, 0,10 bis 0,20 % Na₂HPO₄, 8 bis 15 % Saccharose, 2 bis 8 % CaCO₃ oder 0,1 bis 2 % Hefe, 0,5 % bis 8 % BaSO₄ und 0,5 bis 8 % Chitosan mit einer Viskosität zwischen 10 und 50 mPa.s oder 0,5 bis 8 % Cellulose umfasst, wobei die Prozentsätze Massenprozentsätze in g/100 ml sind.

12. Verwendung einer Zusammensetzung nach einem der vorstehenden Ansprüche zur Behandlung von übergewichtigen Personen, die einen Body-Mass-Index von mindestens 25 kg/m2 aufweisen.

13. Verwendung nach Anspruch 12 zur Behandlung von übergewichtigen Personen mit einem Body-Mass-Index von mindestens 30 kg/m².

14. Verwendung einer Zusammensetzung nach einem der Ansprüche 1 bis 11 zur Abgabe von pharmazeutischen oder ernährungsphysiologischen Wirkstoffen in den Magen einer Person.

15. Set, umfassend eine Zusammensetzung gemäß einem der Ansprüche 1 bis 11 und ein oral assimilierbares Lösungsmittel.

## Claims

1. Hydrogel composition for oral administration comprising:
an alginate polymer that forms a gel in an aqueous solution, in the presence of a cation;
a cation for polymerisation of the alginate polymer in an aqueous solution;
an aqueous solution, in a sufficient quantity;
an agent for dissolving the said alginate polymer in the aqueous solution;
a gelation retardig agent;
a floatation agent for forming bubbles of CO₂ in the hydrogel composition; and
an agent for strengthening the mechanical structure of the hydrogel,
the hydrogel formed by means of the said hydrogel composition being dissolved by means of an orally administered final dissolving agent.

2. Composition according to Claim 1, **characterised in that** the strengthening agent is of the polymeric type, forming macromolecules incorporated into the hydrogel.

3. Composition according to one of Claims 1 or 2, **characterised in that** it further includes a radiopaque agent.

4. Composition according to one of Claims 1, 2 or 3, **characterised in that** the alginate polymer is a sodium alginate polymer, and **in that** the cation is calcium.

5. Composition according to one of the preceding Claims, **characterised in that** the agent for dissolving the alginate polymer in the aqueous solution is sucrose.

6. Composition according to one of the preceding Claims, **characterised in that** the gelation retarding agent is Na₂HPO₄.

7. Composition according to one of the preceding Claims, **characterised in that** the agent for strengthening the mechanical structure of the hydrogel is selected from sorbitol, spermine, chitosan, agarose, sodium dodecyl sulfate, phosphatidylcholine, microcrystalline cellulose.

8. Composition according to one of the preceding Claims, **characterised in that** the radiopaque agent is selected from compounds including barium, for example BaSO₄, and compounds including iodine.

9. Composition according to one of the preceding Claims, **characterised in that** the final dissolving agent is selected from citrates, calcium chelators, for example sodium citrate, citric acid or EDTA.

10. Composition according to one of the preceding Claims, **characterised in that** the floatation agent is CaCO₃, glucono-δ-lactone or a microorganism.

11. Composition according to one of the preceding Claims, **characterised in that** it comprises 0.5 to 5% sodium alginate with a viscosity of between 20-200 mPa.s, 1 to 3% CaSO₄, 0.10 to 0.20% Na₂HPO₄, 8 to 15% sucrose, 2 to 8% CaCO₃ or 0.1 to 2% yeast, 0.5% to 8% BaSO₄, and 0.5 to 8% chitosan with a viscosity of between 10 and 50 mPa.s or 0.5 to 8% cellulose, the percentages being weight percentages given in g/100 ml.

12. Use of a composition according to one of the preceding Claims for the treatment of overweight individuals with a Body Mass Index greater than or equal to 25 kg/m².

13. Use according to Claim 12 for the treatment of obese individuals with a Body Mass Index greater than or equal to 30 kg/m².

14. Use of a composition according to one of Claims 1 to 11 for delivering pharmaceutical or nutritional ingredients into the stomach of an individual.

15. A kit comprising a composition according to one of Claims 1 to 11 and an orally administered dissolving agent.
